# EUROPEAN PATENT SPECIFICATION

(11) **EP 2 861 291 B1**
(45) Date of publication and mention of the grant of the patent: **06.01.2021**
(21) Application number: 13742498.2
(22) Date of filing: 27.05.2013
(51) Int. Cl.: A61M 25/06, A61M 5/32

(54) **NEEDLE GUARD**
NADELSCHUTZ
GAINE D'AIGUILLE

(30) Priority: 19.06.2012 IN DE18832012
(43) Date of publication of application: 22.04.2015
(73) Proprietor: Poly Medicure Limited, Faridabad, Haryana 121004 (IN)
(72) Inventor: BAID, Rishi, New Delhi 110048 (IN)
(74) Representative: Thum, Bernhard
(86) International application number: PCT/IB2013/054368
(87) International publication number: WO 2013/190407

(56) References cited:
- EP-A1- 2 517 751
- WO-A1-03/011381
- WO-A1-2011/154767
- WO-A1-2013/124765

## Description

### CROSS-REFERENCE TO THE RELATED APPLICATION

This application claims priority from Indian Patent Application No. 1883/DEL/2012 dated June 19, 2012.

The invention relates to a needle guard for use in a medical device, in particular for use in a catheter apparatus. The needle guard includes a base portionhaving a needle passage extending in an axial direction from a proximal side of the base portion through the base portion to a distal side of the base portion. The needle guard further includes first and second arms extending substantially in the axial direction from the distal side of the base portion and a distal wall which is transversely arranged at a distal region of the first arm.

Such needle guards are generally known and are used to cover the tip of a needle of a medical device after use of the medical device. Typically, needle guards are devised to automatically cover the needle tip after withdrawal of the needle, for example, from a patient. The needle guard thereby serves to prevent accidental pricking of, for example, a medical practitioner by the needle tip after removal of the needle from the medical device. Thereby the needle can be safely disposed of after use, without the danger of transmitting possibly highly infectious and/or deadly diseases to the medical practitioner from the patient. WO 03/011381 A1 discloses a protective device for a needle, whereby the protective device comprises protective means which slidably cooperate with the needle, characterized in that said protective means comprises a combination of safety means having at least one part which, upon retraction of the needle through the protective means, is placed in front of the needle point and prevents the re-use of the needle and blocking means, which cooperate with the safety means and which, when the protective device passes from a non-operative state into an operative state, release said safety means from a blocked position into an unblocked position. EP 2 517 751 A1 discloses a safety IV catheter assembly which includes a catheter assembly, a needle assembly and a needle guard assembly. The needle guard assembly includes a needle guard, a biasing member and a bushing. The bushing is slidably positioned about the needle and includes an inner diameter which is smaller than an enlarged diameter portion of the needle such that the needle cannot be withdrawn through the bore of the bushing. WO 2013/124765 A1 discloses an intravenous catheter apparatus comprising a needle having a needle shaft, a needle tip at the distal end of the needle shaft and a needle hub mounted to the proximal end of the needle shaft, an intravenous catheter tube mounted to a catheter hub and a needle guard movable on the needle shaft, wherein the needle guard comprises: a base portion having a needle passage extending in an axial direction from a proximal side of the base portion through the base portion to a distal side of the base portion, first and second arms extending substantially in the axial direction from the distal side of the base portion and a distal wall which is transversely arranged at a distal region of the first arm, wherein a recess provided in the needle guard receives a stopping element for stopping movement of the needle shaft relative to the needle guard.

See further WO2011154767 A1.

Generally speaking, the term proximal refers to a region of the device or a location on the device which is closest to, for example, a clinician using the device. In contrast to this, the term distal refers to a region of the device which is farthest from the clinician, for example, the distal region of a needle will be the region of a needle containing the needle tip which is to be inserted e.g. into a patient's vein.

It is an object of the invention to provide an improved needle guard.

This object is satisfied by a needle guard in accordance with claim 1.

The needle guard of the present invention includes a base portion made of a first material and having a needle passage which extends in an axial direction from a proximal side of the base portion through the base portion to a distal side of the base portion, such that a needle having a principal outer profile can be movably arranged in the needle passage. The needle guard further includes first and second arms extending substantially in the axial direction from the distal side of the base portion, with the first arm having a distal region and a proximal region. A distal wall is transversely arranged in the distal region of the first arm.

The needle guard also includes a stopping element which is slidably arranged on the needle shaft and movable relative to the base portion. The stopping element is adapted to engage with an enlargement of the needle shaft on its one side and with the base portion on its opposite side and, thus, effectively helps to prevent the needle guard from sliding beyond the needle tip, i.e. from being separated from the needle.

Because of the movability of the stopping element relative to both the needle and the base portion, the force that has to be applied for pulling the needle through the needle guard upon withdrawal of the needle is reduced.

According to an embodiment, the stopping element completely surrounds the needle. The length of the stopping element, i.e. its dimension seen in the axial direction, may vary. As such, the stopping element can, for example, be a disk, a ring, or a tube. According to an alternative embodiment, it is also possible that the stopping element only partly surrounds the needle. In this case, the stopping element could have the shape of a slotted disk, ring, or tube. Furthermore, it has to be understood that outer the stopping element does not have to have a circular outer profile. It is also possible that the outer profile of the stopping element is of non-circular form, for example, of oval or polygonal shape.

In order to prevent movement of the stopping element through the needle passage, a maximum outer dimension of the stopping element seen in a direction transverse to the axial direction is preferably larger than a maximum transverse dimension of the needle passage.

The stopping element preferably has a through-bore with a profile which is adapted to the principal outer profile of the needle shaft. In the case of e.g. circular cross-sections, a diameter of the through-bore can be slightly larger than a principal outer diameter of the needle.

According to an embodiment, the stopping element is arranged in the base portion. For example, the stopping element can be arranged in a cavity or cut out provided in the base portion. Alternatively, the stopping element can be arranged between the first and second arms.

According to a further embodiment, the stopping element is made of a second material different from the first material. Preferably, the second material is of greater hardness and/or stiffness than the first material. For example, the first material could be a plastic material and the second material could consist of a metal, a ceramic or a rubber material, or any other type of material which is stiff and not as easily distorted as the first material.

Needle guards of the above kind are used, for example, in catheter apparatuses. The invention therefore also provides a catheter apparatus including a needle guard in accordance with the present invention, with the catheter apparatus further including a catheter tube, a catheter hub and a needle having a needle shaft, a needle tip and a needle hub, wherein the needle shaft has a distal section and a proximal section, with at least the proximal section having a principal outer profile.

The needle also has an enlargement near its tip, more specifically between the distal section and the proximal section of the needle shaft. The enlargement has an outer profile one dimension of which is larger than a maximum dimension of the profile of the through-bore of the stopping element. In a preferred embodiment, the enlargement is made by a crimping of the needle shaft. However, other ways of forming the enlargement are possible, such as applying additional material to the needle shaft, e.g. by soldering, welding or gluing etc.

The inner profile of the needle can either be reduced in the region of the enlargement, for example, if the enlargement is formed by crimping, or it can be substantially constant throughout the length of the needle, for example, if the enlargement is formed by applying additional material to the needle shaft.

Prior to the use of the catheter apparatus, the needle guard is arranged in the catheter hub near a proximal end of the needle shaft. In this situation, the needle extends completely through the needle guard, thereby deflecting the first arm of the needle guard outwards, i.e. at an angle to the axial direction, such that the distal wall of the first arm is supported on the needle shaft. Following the insertion of the catheter into a patient, the needle is withdrawn from the catheter tube and the needle shaft moves through the needle guard while the needle guard is retained in the catheter hub. Once the needle tip passes the transverse distal wall of the needle guard, i.e. such that the needle shaft no longer supports the distal wall, a restoring force ensures that the first arm of the needle guard is moved back into alignment with the axial direction of the needle guard, so that the needle tip is blocked by the distal wall of the needle guard, i.e. the needle tip is prevented from axially projecting out of the needle guard.

Once the needle tip is blocked by the distal wall, the enlargement of the needle shaft engages with the stopping element, when the stopping element is arranged between the arms, or with the distal side of the base portion, when the stopping element is arranged in the base portion, to prevent the needle guard from being removed from the needle shaft. The fact that the stopping element is made from a second material which is harder and less easily distorted than the first material of the base portion, has the effect that the needle guard is secured more effectively on the needle shaft and can be retained even if excessive external force is applied when pulling on the needle, as the enlargement is prevented from being pulled through the base portion of the needle guard due to the stopping element. Hence, it is less likely that the needle guard is removed from the needle tip accidentally and, as a result, the needle guard provides a better protection against accidental pricking and thus increased safety for the person handling the catheter apparatus.

In a further embodiment of the needle guard, a tension element surrounds the first and second arms of the needle guard. In the deflected state of the first arm, the tension element is expanded against a restoring force of the tension element. Once the needle shaft no longer supports the distal wall, the tension element aids the repositioning of the first arm back into axial alignment with the axial direction. This repositioning is necessary so that the distal wall can block the needle tip from axially sliding out of the needle guard. In addition, the tension element helps to enclose a space between the first and second arms and thus helps to prevent the needle tip from projecting sideways out of the needle guard. In other words, the tension element adds to the protective effect of the needle guard.

In a further embodiment of the needle guard, a recess is provided in the proximal region of the first arm of the needle guard. This recess increases the deflectability of the first arm in the region it is provided and thereby reduces the restoring force acting on the distal wall while this is being supported by the needle shaft. This allows the needle shaft to be moved more easily relative to the distal wall, as the frictional force acting on the needle shaft is reduced.

In the needle guard, a groove is provided in a side of the distal wall, with the groove extending substantially in the axial direction. The groove acts as a guide groove for the needle shaft and aids the axial movement of the needle shaft relative to the needle guard. Moreover, the needle shaft is prevented from sliding sideways off the distal wall. Such a sideways movement would significantly increase the force required to move the needle shaft relative to the needle guard, which would prevent a correct functioning of the needle guard.

In the needle guard, at least one L-shaped extension extends from the base portion, with a section of the L-shaped extension extending generally in the axial direction. Preferably, two L-shaped extensions are provided on opposite sides of the base portion. The at least one L-shaped extension engages with an outer surface of the catheter hub and helps to retain the needle guard in the catheter hub prior to use of the catheter, thus preventing the needle guard from being removed from the catheter hub before the needle tip is safely received inside the needle guard.

Further advantageous embodiments of the invention and preferred apparatuses for carrying out the invention are set forth in the subordinate claims and are described in connection with the accompanying drawings.

The present invention will now be explained in more detail in the following with reference to preferred embodiments and to the accompanying drawings in which are shown:
Fig. 1 a longitudinal section of a catheter apparatus comprising a needle guard according to a first embodiment of the invention;
Fig. 2 a detail of Fig. 1;
Fig. 3 a side view of the needle guard of Fig. 1 and 2; and
Fig. 4 a side view of a needle guard according to a second embodiment of the invention.

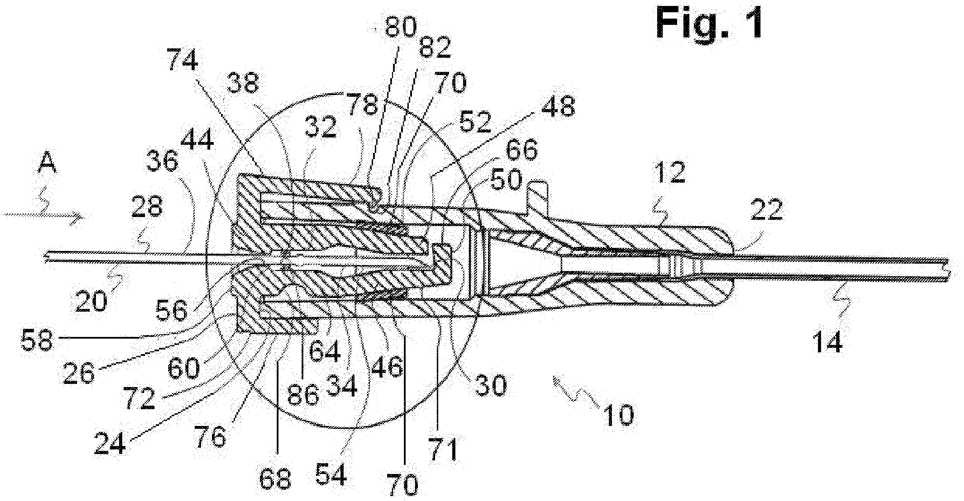

Fig. 1 shows a catheter apparatus 10 in accordance with the invention. The catheter apparatus 10 includes a catheter hub 12, a catheter tube 14 and a needle 20. The catheter hub 12 has a distal end 22 and a proximal end 24, the catheter tube 14 is arranged adjacent to the distal end 22 of the catheter hub 12.

The needle 20 has a needle shaft 28, a needle tip 30 at a distal section 34 of the needle shaft 28 and a needle hub (not shown) attached to a proximal end 36 of the needle shaft 28. Both, the distal section 34 and the proximal section 36 generally have the same outer profile. In the present embodiment, the distal and proximal sections 34, 36 have circular cross-sections with generally identical outer diameters.

An enlargement 32 of the needle 20 is provided between the distal section 34 and the proximal section 36 of the needle shaft 28. The enlargement 32 has a maximum dimension in a direction transverse to the needle shaft 28, which is greater than the outer diameter of the distal and proximal sections 34, 36. The enlargement 32 can be made, for example, by crimping the needle shaft 28.

Prior to use of the catheter apparatus 10, the needle 20 is received in the catheter hub 12 and catheter tube 14, such that the needle shaft 28 extends through the length of the catheter tube 14.

A needle guard 26 is movably arranged on the needle shaft 28 and retained in the catheter hub 12 prior to use of the catheter apparatus 10.The needle guard 26 has a base portion 44, a first arm 46, a second arm 48 and a distal wall 50. The distal wall 50 is arranged at a distal end of the first arm 46 and extends in a direction transverse to an axial direction A. A tension element 52, for example, a rubber band or the like, surrounds the first and second arms 46, 48.

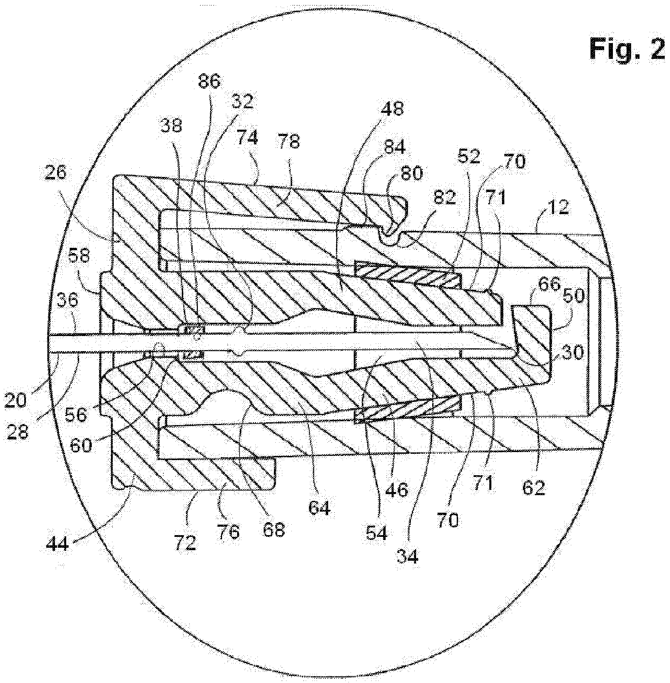

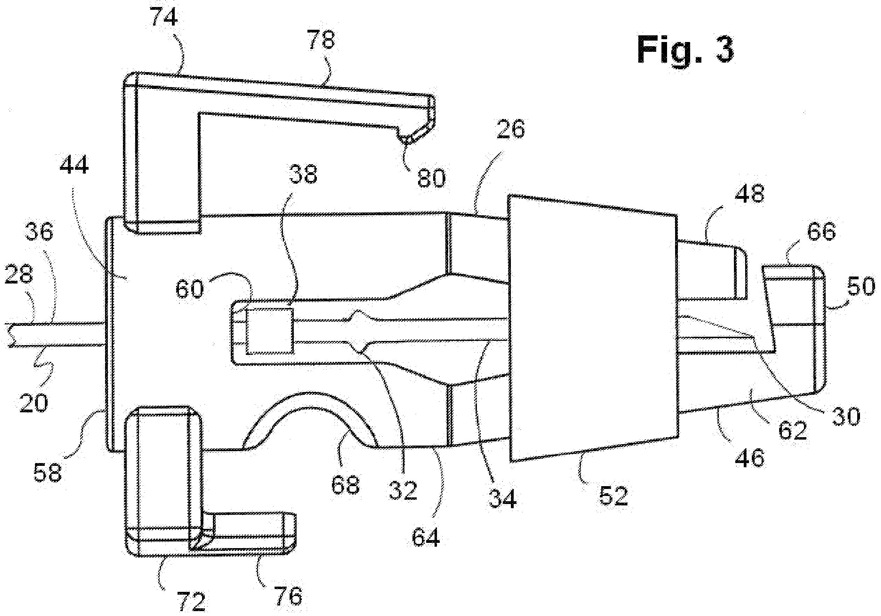

Upon withdrawal of the needle 20 from the catheter tube 14 and catheter hub 12 the needle shaft 28 moves relative to the needle guard 26 until the needle tip 30 is received in the needle guard 26. Once the needle tip 30 is received in the needle guard 26 the enlargement 32 of the needle shaft 28 engages with the base portion 44 of the needle guard 26 via a stopping element 38 such that the needle guard 26 can be pulled out of the catheter hub 12 together with the needle 20. An axial movement of the needle 20 relative to the needle guard 26 is now limited, as the distal wall 50 blocks the needle tip 30 and the engagement between the enlargement 32 and the base portion 44 via the stopping element 38 prevents the needle tip 30 from being removed via the base portion 44, i.e. the needle tip 30 is safely surrounded by the needle guard 26, as is shown in Figs. 1 to 3.

The base portion 44 has a needle passage 56 extending in the axial direction A from a proximal side 58 of the base portion 44 through the base portion 44 to a distal side 60 of the base portion 44. The needle passage 56 is configured to receive the proximal section 36 of the needle shaft 28 and allow movement of the needle shaft 28 relative to the needle guard 26. For this reason, the diameter of the needle passage 56 is slightly larger than the outer diameter of the proximal section 36 of the needle shaft 28.

The first and second arms 46, 48 of the needle guard 26 extend generally in the axial direction A from the distal side 60 of the base portion 44, i.e. generally parallel to the needle shaft 28. The first arm 46 has a distal region 62 and a proximal region 64, with a recess 68 being provided in the proximal region 64 of the first arm 46. The recess 68 is provided to facilitate deflection of the first arm 46 and to reduce a restoring force acting on the first arm 46 when the first arm 46 is deflected off axis.

The outer surfaces 70 of the distal regions 62 of the first and second arms 46, 48 generally taper from the base portion 44 towards the distal wall 50. At their distal ends, the tapered surfaces 70 are limited by shoulders or protrusions 71 formed on the first and second arms 46, 48. The protrusions 71 and the tapered surfaces 70 define the axial position of the tension element 52 and, in particular, prevent the tension element 52 from axially sliding off the first and second arms 46, 48.

The transverse distal wall 50 has a side 66 at its free end, in which a groove (not shown) is provided. The groove extends in a direction generally parallel to the axial direction A and is used to guide the needle shaft 28.

As mentioned above, prior to the use of the catheter apparatus 10 the needle 20 extends through the catheter tube 14 and the needle guard 26 is arranged in the catheter hub 12. In this situation, the distal wall 50 of the needle guard 26 contacts the needle 20, with the needle shaft 28 being guided in the groove in the side 66 of the distal wall 50. The needle shaft 28 thereby supports the distal wall 50, due to which the first arm 46 of the needle guard 26 is deflected outwards, i.e. away from the needle 20, against a restoring force of the tension element 52.

In order to retain the needle guard 26 in the catheter hub 12 while the needle 20 is being withdrawn from the catheter tube 14, the needle guard 26 is provided with first and second L-shaped extensions 72, 74 on opposite sides of the base portion 44.

The first L-shaped extensions 72 comprises a first axial section 76 which extends generally in the axial direction A. The second L-shaped extension 74 comprises a second axial section 78 which is longer than the first axial section 76 and extends slightly off the axial direction A towards the first axial section 76. Prior to use of the catheter apparatus, i.e. when the needle guard is positioned in the catheter hub 12, the first and second axial sections 76, 78 engage with an outer surface of the catheter hub 12. A hook-like protrusion 80 is provided in the region of a distal end of the second axial section 78, which. Prior to use of the catheter apparatus the hook-like protrusion 80 engages with a corresponding combination of locking recession 82 and locking protrusion 84 provided in the outer surface of the catheter hub 12.

Once the needle 20 has been withdrawn such that the needle tip 30 has passed the distal wall 50 and is received between the first and second arms 46, 48, the needle shaft 28 no longer supports the distal wall 50. This causes the first arm 46 to reposition itself in axial alignment with the needle 20 due to the restoring force acting on the first arm 46 in its deflected state. The realignment of the first arm 46 is aided through the use of the tension element 52. The realignment of the first arm 46 results in the needle tip 30 being arranged in a space 54 which is bounded by the base portion 44, the first and second arms 46, 48, the distal wall 50 and the tension element 52.

A stopping element 38 is slidably arranged on the proximal section 36 of the needle shaft 28, such that it can freely move along the needle shaft 28. According to the embodiment shown in Figs. 1 to 3, the stopping element 38 is arranged between the first and second arms 46, 48 of the needle guard 26.

According to the embodiment shown in Figs. 1 to 3, the stopping element 38 has the form of a continuous tube. Alternatively, the stopping element 38 could be a continuous ring or disk, or a discontinuous tube, ring or disk, e.g. slotted tube, ring or disk.

A maximum outer dimension of the stopping element 38 as seen in a direction transverse to the axial direction A is greater than a maximum dimension of the needle passage 56 as seen in the transverse direction. In the present embodiment, the stopping element 38 has a circular outer profile. However, non-circular outer profiles, e.g. oval or polygonal outer profiles would also be possible.

The stopping element 38 is made of a material different from the material of the base portion 44, in particular, a material having a greater hardness and/or stiffness than the material of the base portion 44. Preferably, the stopping element 38 is made of metal or ceramic, but itcan be made out of any other material which is stiff and is not easily bent.

The base portion 44 and first and second arms 46, 48 of the needle guard 26 can be made from a plastic material, for example by a moulding process, with the stopping element 38 placed within the mould prior to the moulding process. The material of the base portion 44 and the first and second arms 46, 48 is different to the material of the stopping element 38.

The stopping element 38 has a through-bore 86 which has a circular cross-section with its diameter being slightly larger than the principle diameter of the proximal section 36 of the needle shaft 28, in order to allow movement of the proximal section 36 of the needle shaft 28 relative to the stopping element 38. At the same time the diameter of the through-bore 86 is smaller than the maximum dimension of the enlargement 32 of the needle shaft 28, in order to prevent the enlargement 32 from passing through the through-bore 86.

When the needle tip enters the needle guard 26 upon withdrawal of the needle 20, theen largement 32 engages with a distal side of the stopping element 38 and forces the stopping element 38 against the distal side 60 of the base portion 44. Since the diameter of the through-bore 82 of the stopping element 38 is smaller than the maximum dimension of the enlargement 32 and the maximum outer dimension of the stopping element 38 is greater than the maximum dimension of the needle passage 56, the stopping element 38 effectively prevents the enlargement 32 of the needle shaft from being pulled through the needle passage 56 of the base portion 44. Thus, the stopping element 38 improves the safety of the needle guard 26.

The pulling force acting on the needle guard 26 via the needle 20 and the stopping element 38 causes the hook-like protrusion 80 provided on the second axial section 78 of the second L-shaped extension 74 to disengage from the locking recession 82 and locking protrusion 84 provided in the outer surface of the catheter hub 12. The needle guard 26 can thus be removed from the catheter hub 12 with the needle tip 30 being safely received in the needle guard 26.

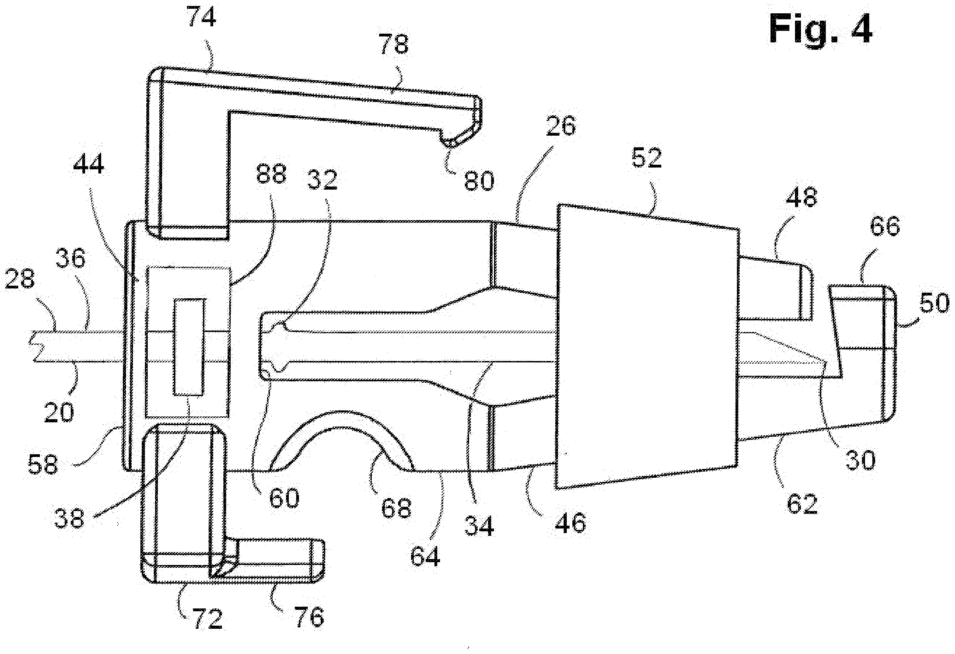

Fig. 4 illustrates a second embodiment of a needle guard 26 which differs from the needle guard 26 according to the above described first embodiment only in the position of the stopping element 38.

In the second embodiment, the stopping element 38 is not arranged between the first and second arms 46, 48, but instead it is arranged in the base portion 44. To this end the base portion 44 is provided with a receptacle 88 for the stopping element 38. As is shown in Fig. 4, the receptacle 88 is formed by a cut out. Alternatively, the receptacle 88 could also be formed by a cavity or chamber in the base portion 44.

An axial dimension of the receptacle 88 is greater than the length of the stopping element 38, such that the stopping element 38 is free to move along the needle shaft 28 within the receptacle 88.

When the needle is withdrawn, the enlargement 32 of the needle shaft 28 normally engages with the distal side 60 of the base portion 44, thereby preventing the needle guard 26 from sliding of the needle 20. Only when excessive pulling forces are applied to the needle 20, the enlargement 32 would be forced through the distal portion of the needle passage 56 in the base portion 44 until it comes into contact with the stopping element 38. The stopping element 38 would then prevent further movement of the enlargement 32 through the base portion 44 and thus ensure that the needle guard 26 stays on the needle and efficiently guards the needle tip 30, thereby improving the protective function of the needle guard 26.

Although this invention has been disclosed in the context of certain preferred embodiments and examples, it will be understood by those skilled in the art that the present invention can be constructed and utilized in a plethora of different ways. It should be understood that many changes, modifications, variations and other uses and applications will become apparent to those persons skilled in this particular area of technology and to others after having been exposed to the present specification and ac companying drawings. Any and all such change, modifications, variations, and other uses and applications which do not depart from the scope of the present invention are therefore covered by and embraced within the present invention and the patent claims set forth herein-below.

### List of Reference Numerals

- 10: catheter
- 12: catheter hub
- 14: catheter tube
- 20: needle
- 22: distal end
- 24: proximal end
- 26: needle guard
- 28: needle shaft
- 30: needle tip
- 32: enlargement
- 34: distal section
- 36: proximal section
- 38: stopping element
- 44: base portion
- 46: first arm
- 48: second arm
- 50: distal wall
- 52: tension element
- 54: space
- 56: needle passage
- 58: proximal side
- 60: distal side
- 62: distal region
- 64: proximal region
- 66: side
- 68: recess
- 70: outer surface
- 71: protrusion
- 72: L-shaped extension
- 74: L-shaped extension
- 76: first axial section
- 78: second axial section
- 80: hook-like protrusion
- 82: locking recession
- 84: locking protrusion
- 86: through-bore
- 88: receptacle
- A: axial direction

## Claims

1. A needle guard (26) for use in a medical device, in particular for use in a catheter device (10), including:
a base portion (44) made of a first material and having a needle passage (56) extending in an axial direction (A) from a proximal side (58) of said base portion (44) through said base portion (44) to a distal side (60) of said base portion (44) for movably receiving a needle shaft (28) having an outer profile;
first and second arms (46, 48) extending substantially in said axial direction (A) from said distal side (60) of said base portion (44), wherein said first arm (46) has a distal region (62) and a proximal region (64);
a distal wall (50) transversely arranged at said distal region (62) of said first arm (46);
a stopping element (38) which is configured to be slidably arranged on a needle shaft (28), a groove configured to guide the needle shaft (28) is provided in a side (66) at the free end of said distal wall (50), said groove extending substantially in said axial direction (A); and
at least one L-shaped extension (72, 74) extends from said base portion (44), a section of said L-shaped extension (72, 74) extending generally in said axial direction (A),
**characterized in that** said stopping element (38) is movable relative to said base portion (44).

2. A needle guard (26) in accordance with claim 1, wherein a maximum outer dimension of said stopping element (38) seen in a direction transverse to said axial direction (A) is larger than a maximum transverse dimension of said needle passage (56).

3. A needle guard (26) in accordance with claim 1 or 2, wherein said stopping element (38) has a through-bore (86) with a profile that is adapted to the outer profile of the needle shaft (28).

4. A needle guard (26) in accordance with any one of the preceding claims, wherein said stopping element (38) is made of a second material different from said first material.

5. A needle guard (26) in accordance with claim 4, wherein said second material is of a greater hardness and/or stiffness than the first material.

6. A needle guard (26) in accordance with any one of the preceding claims, wherein said stopping element (38) is arranged in said base portion (44).

7. A needle guard (26) in accordance with any one of the preceding claims, wherein said stopping element (38) is arranged in a cavity or cut out provided in said base portion (44).

8. A needle guard (26) in accordance with any one of claims 1 to 5, wherein said stopping element (38) is arranged between said first and second arms (46, 48).

9. A needle guard (26) in accordance with any one of the preceding claims, wherein said stopping element (38) has a disk-like, ring-like or tube-like shape.

10. A needle guard (26) in accordance with any one of the preceding claims, wherein a tension element (52) is provided which is arranged such that it surrounds said first and second arms (46, 48) of said needle guard (26).

11. A needle guard (26) in accordance with claim 1, wherein two L-shaped extensions (72, 74) are provided on opposite sides of said base portion (44).

12. A catheter apparatus (40), including:
a catheter tube (14);
a catheter hub (12);
a needle (20) having a needle tip (30) and a needle shaft (28), wherein said needle shaft (28) has a distal section (34) and a proximal section (36), with at least the proximal section (36) having an outer profile; and
a needle guard (26) in accordance with any one of the preceding claims,
wherein said needle shaft (28) has an enlargement (32) between said distal section (34) and said proximal section (36), said enlargement (32) having an increased outer profile a dimension of which is larger than a maximum dimension of the profile of the needle passage (56) and/or the stopping element (38).

## Patentansprüche

1. Nadelschutz (26) zur Verwendung in einer medizinischen Vorrichtung, insbesondere zur Verwendung in einer Kathetervorrichtung (10), umfassend:
einen Basisabschnitt (44) aus einem ersten Material und mit einem Nadeldurchgang (56), der sich in einer axialen Richtung (A) von einer proximalen Seite (58) des Basisabschnitts (44) durch den Basisabschnitt (44) zu einer distalen Seite (60) des Basisabschnitts (44) hin erstreckt, um einen Nadelschaft (28) mit einem Außenprofil beweglich aufzunehmen;
erste und zweite Arme (46, 48), die sich von der distalen Seite (60) des Basisabschnitts (44) aus im Wesentlichen in der axialen Richtung (A) erstrecken, wobei der erste Arm (46) einen distalen Bereich (62) und einen proximalen Bereich (64) aufweist;
eine distale Wand (50), die quer an dem distalen Bereich (62) des ersten Arms (46) angeordnet ist;
ein Stoppelement (38), das dazu eingerichtet ist, an einem Nadelschaft (28) verschiebbar zu sein,
eine Aussparung, die zur Führung des Nadelschafts (28) eingerichtet ist, die in einer Seite (66) am freien Ende der distalen Wand (50) bereitgestellt ist, wobei sich die Aussparung im Wesentlichen in der axialen Richtung (A) erstreckt; und
mindestens einen L-förmigen Fortsatz (72, 74) der sich von dem Basisabschnitt (44) aus erstreckt, wobei sich ein Abschnitt des L-förmigen Fortsatzes (72, 74) im Allgemeinen in der axialen Richtung (A) erstreckt,
**dadurch gekennzeichnet, dass** das Stoppelement (38) in Bezug auf den Basisabschnitt (44) beweglich ist.

2. Nadelschutz (26) gemäß Anspruch 1, wobei eine maximale äußere Ausdehnung des Stoppelements (38), quer zu der axialen Richtung (A) betrachtet, größer als eine maximale Querausdehnung des Nadeldurchgangs (56) ist.

3. Nadelschutz (26) gemäß Anspruch 1 oder 2, wobei das Stoppelement (38) eine Durchgangsbohrung (86) mit einem Profil, das an das Außenprofil des Nadelschafts (28) angepasst ist, aufweist.

4. Nadelschutz (26) gemäß einem beliebigen der vorhergehenden Ansprüche, wobei das Stoppelement (38) aus einem zweiten Material hergestellt ist, das sich von dem ersten Material unterscheidet.

5. Nadelschutz (26) gemäß Anspruch 4, wobei das zweite Material eine größere Härte und/oder Steifigkeit als das erste Material aufweist.

6. Nadelschutz (26) gemäß einem beliebigen der vorhergehenden Ansprüche, wobei das Stoppelement (38) in dem Basisabschnitt (44) angeordnet ist.

7. Nadelschutz (26) gemäß einem beliebigen der vorhergehenden Ansprüche, wobei das Stoppelement (38) in einem in dem Basisabschnitt (44) vorgesehenen Hohlraum oder Ausschnitt angeordnet ist.

8. Nadelschutz (26) gemäß einem beliebigen der Ansprüche 1 bis 5, wobei das Stoppelement (38) zwischen den ersten und zweiten Armen (46, 48) angeordnet ist.

9. Nadelschutz (26) gemäß einem beliebigen der vorhergehenden Ansprüche, wobei das Stoppelement (38) eine scheibenartige, ringartige oder röhrenartige Form hat.

10. Nadelschutz (26) gemäß einem beliebigen der vorhergehenden Ansprüche, wobei ein Spannelement (52) bereitgestellt ist, das so angeordnet ist, dass es die ersten und zweiten Arme (46, 48) des Nadelschutzes (26) umgibt.

11. Nadelschutz (26) gemäß Anspruch 1, wobei zwei L-förmige Fortsätze (72, 74) an gegenüberliegenden Seiten des Basisabschnitts (44) vorgesehen sind.

12. Kathetervorrichtung (40), umfassend:
einen Katheterschlauch (14);
einen Katheteransatz (12);
eine Nadel (20) mit einer Nadelspitze (30) und einem Nadelschaft (28), wobei der Nadelschaft (28) einen distalen Abschnitt (34) und einen proximalen Abschnitt (36) aufweist, wobei zumindest der proximale Abschnitt (36) ein Außenprofil aufweist; und
einen Nadelschutz (26) gemäß einem beliebigen der vorhergehenden Ansprüche,
wobei der Nadelschaft (28) zwischen dem distalen Abschnitt (34) und dem proximalen Abschnitt (36) eine Erweiterung (32) aufweist, wobei die Erweiterung (32) ein vergrößertes Außenprofil aufweist, dessen Ausdehnung größer ist, als eine maximale Ausdehnung des Profils des Nadeldurchgangs (56) und/oder des Stoppelements (38).

## Revendications

1. Garde-aiguille (26) pour une utilisation dans un dispositif médical, en particulier pour une utilisation dans un dispositif (10) de cathéter, incluant :
une partie (44) de base constituée d'une première matière et ayant un passage (56) pour aiguille s'étendant dans un sens axial (A) d'un côté proximal (58) de ladite partie (44) de base à travers ladite partie (44) de base jusqu'à un côté distal (60) de ladite partie (44) de base pour recevoir en mobilité une tige (28) d'aiguille ayant un profil extérieur ;
des premier et deuxième bras (46, 48) s'étendant sensiblement dans ledit sens axial (A) depuis ledit côté distal (60) de ladite partie (44) de base, dans lequel ledit premier bras (46) a une région distale (62) et une région proximale (64) ;
une paroi distale (50) agencée transversalement au niveau de ladite région distale (62) dudit premier bras (46) ;
un élément d'arrêt (38) qui est configuré pour être agencé coulissant sur une tige (28) d'aiguille,
une rainure configurée pour guider la tige (28) d'aiguille est prévue dans un côté (66) à l'extrémité libre de ladite paroi distale (50), ladite rainure s'étendant sensiblement dans ledit sens axial (A) ; et
au moins une extension (72, 74) en forme de L s'étend depuis ladite partie (44) de base, une section de ladite extension (72, 74) en forme de L s'étendant de manière générale dans ledit sens axial (A),
**caractérisé en ce que** ledit élément d'arrêt (38) est mobile par rapport à ladite partie (44) de base.

2. Garde-aiguille (26) selon la revendication 1, dans lequel une dimension extérieure maximum dudit élément d'arrêt (38) vu dans un sens transversal audit sens axial (A) est plus grande qu'une dimension transversale maximum dudit passage (56) pour aiguille.

3. Garde-aiguille (26) selon la revendication 1 ou 2, dans lequel ledit élément d'arrêt (38) a un alésage traversant (86) avec un profil qui est adapté au profil extérieur de la tige (28) d'aiguille.

4. Garde-aiguille (26) selon l'une quelconque des revendications précédentes, dans lequel ledit élément d'arrêt (38) est constitué d'une deuxième matière différente de ladite première matière.

5. Garde-aiguille (26) selon la revendication 4, dans lequel ladite deuxième matière est d'une dureté et/ou d'une rigidité supérieure(s) à la première matière.

6. Garde-aiguille (26) selon l'une quelconque des revendications précédentes, dans lequel ledit élément d'arrêt (38) est agencé dans ladite partie (44) de base.

7. Garde-aiguille (26) selon l'une quelconque des revendications précédentes, dans lequel ledit élément d'arrêt (38) est agencé dans une cavité ou une découpe prévue dans ladite partie (44) de base.

8. Garde-aiguille (26) selon l'une quelconque des revendications 1 à 5, dans lequel ledit élément d'arrêt (38) est agencé entre lesdits premier et deuxième bras (46, 48).

9. Garde-aiguille (26) selon l'une quelconque des revendications précédentes, dans lequel ledit élément d'arrêt (38) a une forme de type disque, de type bague ou de type tube.

10. Garde-aiguille (26) selon l'une quelconque des revendications précédentes, dans lequel un élément de tension (52) est prévu, qui est agencé de telle manière qu'il entoure lesdits premier et deuxième bras (46, 48) dudit garde-aiguille (26).

11. Garde-aiguille (26) selon la revendication 1, dans lequel deux extensions (72, 74) en forme de L sont prévues sur des côtés opposés de ladite partie (44) de base.

12. Appareil (40) de cathéter, incluant :
un tube (14) de cathéter ;
un pavillon (12) de cathéter ;
une aiguille (20) ayant une pointe (30) d'aiguille et une tige (28) d'aiguille, dans lequel ladite tige (28) d'aiguille a une section distale (34) et une section proximale (36), avec au moins la section proximale (36) ayant un profil extérieur ; et
un garde-aiguille (26) selon l'une quelconque des revendications précédentes,
dans lequel ladite tige (28) d'aiguille a un élargissement (32) entre ladite section distale (34) et ladite section proximale (36), ledit élargissement (32) ayant un profil extérieur augmenté dont une dimension est plus grande qu'une dimension maximum du profil du passage (56) pour aiguille et/ou de l'élément d'arrêt (38).
